# EUROPEAN PATENT SPECIFICATION

(11) **EP 0 865 294 B1**
(45) Date of publication and mention of the grant of the patent: **18.02.2004**
(21) Application number: 96926873.9
(22) Date of filing: 02.08.1996
(51) Int. Cl.: A61K 38/17, A61P 3/02

(54) **METHODS OF REDUCING OR MAINTAINING REDUCED LEVELS OF BLOOD LIPIDS USING OB PROTEIN COMPOSITIONS**
VERFAHREN ZUR VERRINGERUNG UND ZUR ERHALTUNG VON VERRINGERTEN BLUTSPIEGELN VON LIPIDEN MITTELS ZUBEREITUNGEN VON OB-PROTEINEN
PROCEDES POUR DIMINUER OU MAINTENIR UN NIVEAU BAS DE LIPIDES DANS LE SANG, EN UTILISANT DES COMPOSITIONS DE PROTEINE OB

(30) Priority: 17.08.1995 US 516263
(43) Date of publication of application: 23.09.1998
(73) Proprietor: Amgen Inc., Thousand Oaks, CA 91320-1799 (US)
(72) Inventor: PELLEYMOUNTER, Mary, Ann, San Diego, CA 92130-1867 (US)
(74) Representative: Brown, John David
(86) International application number: PCT/US1996/012674
(87) International publication number: WO 1997/006816

(56) References cited:
- WO-A-96/05309
- JOURNAL OF CLINICAL INVESTIGATION, vol. 96, no. 3, 1 September 1995, pages 1647-1652, XP000571403 OGAWA Y ET AL: "MOLECULAR CLONING OF RAT OBESE CDNA AND AUGMENTED GENE EXPRESSION IN GENETICALLY OBESE ZUCKER FATTY (FA/FA) RATS"
- JOURNAL OF BIOLOGICAL CHEMISTRY, vol. 271, no. 16, 19 April 1996, pages 9455-9459, XP000601968 ZHANG B ET AL: "DOWN-REGULATION OF THE EXPRESSION OF THE OBESE GENE BY AN ANTIDIABETIC THIAZOLIDINEDIONE IN ZUCKER DIABETIC FATTY RATS AND DB/DB MICE"
- NATURE, vol. 372, no. 6505, 1 December 1994, LONDON GB, pages 425-432, XP002003607 Y. ZHANG ET AL.: "Positional cloning of the mouse obese gene and its human homologue"

### SEQUENCE LISTING

(1) GENERAL INFORMATION:
   (i) APPLICANT: Amgen Inc.
   (ii) TITLE OF INVENTION: METHODS OF REDUCING OR MAINTAINING REDUCED LEVELS OF BLOOD LIPIDS USING OB PROTEIN COMPOSITIONS
   (iii) NUMBER OF SEQUENCES: 4
   (iv) CORRESPONDENCE ADDRESS:
      (A) ADDRESSEE: Amgen Inc.
      (B) STREET: 1840 Dehavilland Drive
      (C) CITY: Thousand Oaks
      (D) STATE: California
      (E) COUNTRY: USA
      (F) ZIP: 91230-1789
   (v) COMPUTER READABLE FORM:
      (A) MEDIUM TYPE: Floppy disk
      (5) COMPUTER: IBM PC compatible
      (C) OPERATING SYSTEM: PC-DOS/MS-DOS
      (D) SOFTWARE: Patent In Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Pessin, Karol M.
      (C) REFERENCE/DOCKET NUMBER: A-355
(2) INFORMATION FOR SEQ ID NO:1:
   (i) SEQUENCE CHARACTERISTICS:
      (A) LENGTH: 491 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 41..481
   (xi) SEQUENCE DESCRIPTION: SEQ ID NO:1:
   TCTAGATTTG AGTTTTAACT TTTAGAAGGA GGAATAACAT ATG GTA CCG ATC CAG      55
                                               Met Val Pro Ile Gln
                                                1               5
   AAA GTT CAG GAC GAC ACC AAA ACC TTA ATT AAA ACG ATC GTT ACG CGT     103
   Lys Val Gln Asp Asp Thr Lys Thr Leu Ile Lys Thr Ile Val Thr Arg
                    10                  15                  20
   ATC AAC GAC ATC AGT CAC ACC CAG TCG GTC TCC GCT AAA CAG CGT GTT     151
   Ile Asn Asp Ile Ser His Thr Gln Ser Val Ser Ala Lys Gln Arg Val
               25                  30                  35
   ACC GGT CTG GAC TTC ATC CCG GGT CTG CAC CCG ATC CTA AGC TTG TCC     199
   Thr Gly Leu Asp Phe Ile Pro Gly Leu His Pro Ile Leu Ser Leu Ser
           40                  45                  50
   AAA ATG GAC CAG ACC CTG GCT GTA TAC CAG CAG GTG TTA ACC TCC CTG     247
   Lys Met Asp Gln Thr Leu Ala Val Tyr Gln Gln Val Leu Thr Ser Leu
       55                  60                  65
   CCG TCC CAG AAC GTT CTT CAG ATC GCT AAC GAC CTC GAG AAC CTT CGC     295
   Pro Ser Gln Asn Val Leu Gln Ile Ala Asn Asp Leu Glu Asn Leu Arg
    70                  75                  80                  85
   GAC CTG CTG CAC CTG CTG GCA TTC TCC AAA TCC TGC TCC CTG CCG CAG     343
   Asp Leu Leu His Leu Leu Ala Phe Ser Lys Ser Cys Ser Leu Pro Gln
                    90                  95                 100
   ACC TCA GGT CTT CAG AAA CCG GAA TCC CTG GAC GGG GTC CTG GAA GCA     391
   Thr Ser Gly Leu Gln Lys Pro Glu Ser Leu Asp Gly Val Leu Glu Ala
               105                 110                 115
   TCC CTG TAC AGC ACC GAA GTT GTT GCT CTG TCC CGT CTG CAG GGT TCC     439
   Ser Leu Tyr Ser Thr Glu Val Val Ala Leu Ser Arg Leu Gln Gly Ser
           120                 125                 130
   CTT CAG GAC ATC CTT CAG CAG CTG GAC GTT TCT CCG GAA TGT             481
   Leu Gln Asp Ile Leu Gln Gln Leu Asp Val Ser Pro Glu Cys
       135                 140                 145
   TAATGGATCC                                                          491
(2) INFORMATION FOR SEQ ID NO:2:
   (i) SEQUENCE CHARACTERISTICS:
      (A) LENGTH: 147 amino acids.
      (B) TYPE: amino acid
      (D) TOPOLOGY: linear
   (ii) MOLECULE TYPE: protein
   (xi) SEQUENCE DESCRIPTION: SEQ ID NO:2:
   Met Val Pro Ile Gln Lys Val Gln Asp Asp Thr Lys Thr Leu Ile Lys
    1               5                  10                  15
   Thr Ile Val Thr Arg Ile Asn Asp Ile Ser His Thr Gln Ser Val Ser
               20                  25                  30
   Ala Lys Gln Arg Val Thr Gly Leu Asp Phe Ile Pro Gly Leu His Pro
           35                  40                  45
   Ile Leu Ser Leu Ser Lys Met Asp Gln Thr Leu Ala Val Tyr Gln Gln
       50                  55                  60
   Val Leu Thr Ser Leu Pro Ser Gln Asn Val Leu Gln Ile Ala Asn Asp
   65                  70                  75                  80
   Leu Glu Asn Leu Arg Asp Leu Leu His Leu Leu Ala Phe Ser Lys Ser
                   85                  90                  95
   Cys Ser Leu Pro Gln Thr Ser Gly Leu Gln Lys Pro Glu Ser Leu Asp
               100                 105                 110
   Gly Val Leu Glu Ala Ser Leu Tyr Ser Thr Glu Val Val Ala Leu Ser
           115                 120                 125
   Arg Leu Gln Gly Ser Leu Gln Asp Ile Leu Gln Gln Leu Asp Val Ser
       130                 135                 140
   Pro Glu Cys
   145
(2) INFORMATION FOR SEQ ID NO:3:
   (i) SEQUENCE CHARACTERISTICS:
      (A) LENGTH: 454 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 4..444
   (xi) SEQUENCE DESCRIPTION: SEQ ID NO:3:
   CAT ATG GTA CCG ATC CAG AAA GTT CAG GAC GAC ACC AAA ACC TTA ATT          48
       Met Val Pro Ile Gln Lys Val Gln Asp Asp Thr Lys Thr Leu Ile
        1              5                  10                  15
   AAA ACG ATC GTT ACG CGT ATC AAC GAC ATC AGT CAC ACC CAG TCG GTG          96
   Lys Thr Ile Val Thr Arg Ile Asn Asp Ile Ser His Thr Gln Ser Val
                    20                  25                  30
   AGC TCT AAA CAG CGT GTT ACA GGC CTG GAC TTC ATC CCG GGT CTG CAC         144
   Ser Ser Lys Gln Arg Val Thr Gly Leu Asp Phe Ile Pro Gly Leu His
                35                  40                  45
   CCG ATC CTG ACC TTG TCC AAA ATG GAC CAG ACC CTG GCT GTA TAC CAG         192
   Pro Ile Leu Thr Leu Ser Lys Met Asp Gln Thr Leu Ala Val Tyr Gln
            50                  55                  60
   CAG ATC TTA ACC TCC ATG CCG TCC CGT AAC GTT CTT CAG ATC TCT AAC         240
   Gln Ile Leu Thr Ser Met Pro Ser Arg Asn Val Leu Gln Ile Ser Asn
        65                  70                  75
   GAC CTC GAG AAC CTT CGC GAC CTG CTG CAC GTG CTG GCA TTC TCC AAA         288
   Asp Leu Glu Asn Leu Arg Asp Leu Leu His Val Leu Ala Phe Ser Lys
    80                  85                  90                  95
   TCC TGC CAC CTG CCA TGG GCT TCA GGT CTT GAG ACT CTG GAC TCT CTG         336
   Ser Cys His Leu Pro Trp Ala Ser Gly Leu Glu Thr Leu Asp Ser Leu
                   100                 105                 110
   GGC GGG GTC CTG GAA GCA TCC GGT TAC AGC ACC GAA GTT GTT GCT CTG         384
   Gly Gly Val Leu Glu Ala Ser Gly Tyr Ser Thr Glu Val Val Ala Leu
               115                 120                 125
   TCC CGT CTG CAG GGT TCC CTT CAG GAC ATG CTT TGG CAG CTG GAC CTG         432
   Ser Arg Leu Gln Gly Ser Leu Gln Asp Met Leu Trp Gln Leu Asp Leu
           130                 135                 140
   TCT CCG GGT TGT TAATGGATCC                                              454
   Ser Pro Gly Cys
       145
(2) INFORMATION FOR SEQ ID No:4:
   (i) SEQUENCE CHARACTERISTICS:
      (A) LENGTH: 147 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Val Pro Ile Gln Lys Val Gln Asp Asp Thr Lys Thr Leu Ile Lys
   1 5 10 15
   Thr Ile Val Thr Arg Ile Asn Asp Ile Ser His Thr Gln Ser Val Ser
   20 25 30
   Ser Lys Gln Arg Val Thr Gly Leu Asp Phe Ile Pro Gly Leu His Pro
   35 40 45
   Ile Leu Thr Leu Ser Lys Met Asp Gln Thr Leu Ala Val Tyr Gln Gln
   50 55 60
   Ile Leu Thr Ser Met Pro Ser Arg Asn Val Leu Gln Ile Ser Asn Asp
   65 70 75 80
   Leu Glu Asn Leu Arg Asp Leu Leu His Val Leu Ala Phe Ser Lys Ser
   85 90 95
   Cys His Leu Pro Trp Ala Ser Gly Leu Glu Thr Leu Asp Ser Leu Gly
   100 105 110
   Gly Val Leu Glu Ala Ser Gly Tyr Ser Thr Glu Val Val Ala Leu Ser
   115 120 125
   Arg Leu Gln Gly Ser Leu Gln Asp Met Leu Trp Gln Leu Asp Leu Ser
   130 135 140
   Pro Gly Cys
   145

## Description

### FIELD OF THE INVENTION

The present invention relates to the use of an OB protein, analog or derivative thereof for the manufacture of a medicament for reducing or maintaining, reduced levels of blood lipids in a non-obese patient. The present invention is for the treatment of high cholesterol, high triglyceride levels, arterial plaque, hypertension, and prevention of gall stone formation.

### BACKGROUND

Although the molecular basis for obesity is largely unknown, the identification of the "OB gene" and protein encoded ("OB protein") has shed some light on mechanisms the body uses to regulate body fat deposition. Zhang et al., Nature 372: 425-432 (1994); see also, the Correction at Nature 374: 479 (1995). The OB protein is active in vivo in both ob/ob mutant mice (mice obese due to a defect in the production of the OB gene product) as well as in normal, wild type mice. The biological activity manifests itself in, among other things, weight loss. See generally, Barinaga, "Obese" Protein Slims Mice, Science 269: 475-476 (1995).

The other biological effects of OB protein are not well characterized. It is known, for instance, that in ob/ob mutant mice, administration of OB protein results in a decrease in serum insulin levels, and serum glucose levels. It is also known that administration of OB protein in ob/ob mutant mice results in a decrease in body fat. Pelleymounter et al., Science 269:540-543 (1995); Halaas et al., Science 269: 543-546 (1995). This was observed in both ob/ob mutant mice, as well as non-obese normal mice. Halaas et al., supra; see also, Campfield et al., Science 269: 546-549 (1995)(Peripheral and central administration of microgram doses of OB protein reduced food intake and body weight of ob/ob and diet-induced obese mice but not in db/db obese mice.) In none of these reports have toxicities been observed, even at the highest doses.

The elucidation of other biological effects of the OB protein, particularly on animals which may not benefit from or may not need weight reduction, will provide additional uses for the OB protein.

One such use, as provided by the present invention, is in cardio-vascular therapies.

Presently, drugs used to treat blood lipids have side effects to varying degrees. (The summary below is fully explained in Remington's Pharmaceutical Sciences, 18th Ed. (1990, Mack Publishing Co., Easton, PA 18042) Chapter 41, at pages 855-859.))

Cholestyramine resin and Colestipol Hydrocholoride are used to bind bile acids in the intestine and, hence, to prevent their absorption in hypercholesterolemias. Noted side effects include constipation (20-50%), heartburn and dyspepsia, colic, belching, bloating, biliary stasis and lodged gallstones, steatorrhea and malapsorption syndrome (with doses >24 g/day) and consequent hypovitaminosis A, D and K. Other side effects are also noted.

Clofibrate is used to decrease very low density lipoprotein ("VLDL") in persons with hypertriglyceridemia. The mode of action is thought to be that of suppressing release of free fatty acids from fat cells. Side effects include nausea, dyspepsia, diarrhea, stomatitis and flatulence (in 10% of patients), urticaria, pruritus and stomatitis, alopecia, headache, vertigo, asthenia, myalgia, dermatitis, slight weight gain, breast tenderness in males, decrease in libido, impotence, and dry and brittle hair in women occur with varying degrees of frequency. The incidence of cholesterolic gallstones was noted to increase twofold. Other side effects are also noted.

Gemfibrozil is used to decrease the hepatic synthesis of VLDL. Side effects include abdominal pain, epigastric pain, diarrhea, nausea, vomiting, and flatulence.

Lovastatinis used to inhibit an enzyme which is necessary in the synthesis of cholesterol. Side effects are usually mild and transient, and include headache, flatus, abdominal pain/cramps, diarrhea, rash/pruritis, constipation, nausea, myalgia, dizziness, blurred vision, muscle cramps, and dysgeusia. Sleep abnormalities are noted to be frequent.

Probucol is used to lower plasma low density lipoprotein ("LDL") and cholesterol by decreasing cholesterol synthesis at an early stage, increasing the catabolism of LDL and increasing the excretion of bile acids. Side effects include diarrhea, transient flatulence, abdominal pain, nausea, hyperhidrosis, fetid sweat, vomiting, dizziness, chest pain, and palpitations.

It would therefore be useful to have a therapeutic composition which modulates blood lipid levels, particularly in non-obese patients, without side effects seen in the presently available drugs. Such composition would have utility in the treatment of high cholesterol levels, high triglyceride levels, arterial plaque, and relatedly hypertension, and gall stone formation.

### SUMMARY OF THE INVENTION

The present invention stems from the observation that administration of OB protein to obese animals having elevated cholesterol levels results in a reduction of serum cholesterol levels to normal levels. Thus, OB protein has the capacity to act, in addition to acting as a weight reducing agent, as an agent affecting blood lipids. As such, numerous cardio-vascular therapies are contemplated, even for patients who would not necessarily benefit from weight reduction.

There is a positive relationship between blood lipid levels, particularly cholesterol levels and triglyceride levels, and arteriosclerosis, as well as coronary occlusion. E.g., Remington's Pharmaceutical Sciences, 18th Ed. (1990, Mack Publishing Co., Easton, PA 18042) Chapter 41, at pages 855-859. Some studies show that a low-fat diet in combination with antilipidemic drugs have a protective effect in coronary heart disease. Reduction of blood lipid levels would have tremendous therapeutic effect in the cardio-vascular area, even in the absence of concomitant weight loss.

For example; administration of OB protein (or analogs or derivatives thereof) are useful to reduce serum cholesterol, and triglycerides in patients having elevated levels of these blood lipids.

Thus, one aspect of the present invention is for use in treating a non-obese animal for elevated blood lipid levels.

More particularly, the present invention is useful for reducing the blood cholesterol in a non-obese patient. Belatedly, when an individual is treated for elevated cholesterol levels, particularly a patient with familial hypercholesterolemia, the incidence of xanthomas may be diminished.

The present invention is also useful for the treatment to reduce or maintain reduced triglyceride levels in a non-obese patient.

Relatedly, another aspect of the present invention is for use in treating an obese or non-obese animal for reducing or preventing the formation of arterial plaque. Arterial plaque is comprised, in large part, of fat and cholesterol. Reduction in blood lipid levels, resulting in reduction of cholesterol levels would result in reduction of arterial plaque. Reduction in arterial plaque may also result in an improvement in atherosclerosis.

In addition, reduction in arterial plaque may increase blood flow, and therefore reduce the blood pressure, such as in systolic hypertension. Thus, another aspect of the present invention is the use of OB protein, or analogs or derivatives thereof to manufacture a medicament, as an antihypertensive agent in the treatment of high blood pressure.

Furthermore, reduction in blood lipids may benefit those experiencing gall stones. Gall stones are formed as a result of improper bile processing in the gall bladder. Bile is rich in fatty substances, especially cholesterol, that are extracted from the blood by the liver. Bile also contains bilirubin, a substance that if formed by the breakdown of hemoglobin from old red blood cells. If the balance of these substances is upset, a tiny solid particle forms in the gall bladder. The particle may grow as more material solidifies around it. Thus, in yet another aspect, the present invention provides the use of OB protein, or analogs or derivatives thereof to manufacture a medicament to prevent the formation of gall stones or reduce the formation of additional gall stones.

Also contemplated herein is a course of therapy wherein OB protein (or analog or derivative thereof) is to be administered to a non-obese patient in dosages sufficient for reduction in blood lipid level (as compared to levels in the absence of OB protein administration), but such dosages do not result in weight loss (or further weight loss if the patient has previously been administered OB protein or analog or derivative thereof) for weight reduction.

### DETAILED DESCRIPTION

The uses underlying the present invention are those directed to the treatment of a non-obese individual for reduction in blood lipid level by administration of OB protein, or analogs, or derivatives thereof. These uses include methods for reducing or maintaining reduced blood cholesterol levels, blood triglyceride levels (i.e., low density lipoprotein (LDL) or very low density lipoprotein (VLDL)), and methods for reducing arterial plaque presence and formation. The reduction in blood lipids will likely have a beneficial effect on atherosclerosis, and may increase blood flow, and thus reduce hypertension. In addition, the formation of gall stones, which are composed largely of cholesterol, may be prevented.

The OB protein may be selected from the recombinant murine set forth below (SEQ. ID No. 2), or the recombinant human protein as set forth in Zhang et al., Nature, supra) or those lacking a glutaminyl residue at position 28. (See Zhang et al, Nature, supra, at page 428.) One may also use the recombinant human OB protein analog as set forth in SEQ.ID.NO. 4, which contains 1) an arginine in place of lysine at position 35 and 2) a leucine in place of isoleucine at position 74. (A shorthand abbreviation for this analog is the recombinant human R->L³⁵, I->L⁷⁴). The amino acid sequences for the recombinant human analog and recombinant murine proteins are set forth below with a methionyl residue at the -1 position however, as with any of the present OB proteins and analogs, the methionyl residue may be absent.

The murine protein is substantially homologous to the human protein, particularly as a mature protein, and, further, particularly at the N-terminus. One may prepare an analog of the recombinant human protein by altering (such as substituting amino acid residues), in the recombinant human sequence, the amino acids which diverge from the murine sequence. Because the recombinant human protein has biological activity in mice, such analog would likely be active in humans. For example, using a human protein having a lysine at residue 35 and an isoleucine at residue 74 according to the numbering of SEQ. ID NO. 4, wherein the first amino acid is valine, and the amino acid at position 146 is cysteine, one may substitute with another amino acid one or more of the amino acids at positions 32, 35, 50, 64, 68, 71, 74, 77, 89, 97, 100, 105, 106, 107, 108, 111, 118, 136, 138, 142, and 145. One may select the amino acid at the corresponding position of the murine protein, (SEQ. ID. NO. 2), or another amino acid.

One may further prepare "consensus" molecules based on the rat OB protein sequence. Murakami et al., Biochem.Biophys.Res. Comm. 209: 944-952 (1995). Rat OB protein differs from human OB protein at the following positions (using the numbering of SEQ. ID. NO. 4): 4, **32,** 33, **35, 50,** 68, **71, 74, 77,** 78, **89**, **97**, **100,** 101, 102, **105, 106, 107, 108, 111, 118, 136, 138** and **145.** One may substitute with another amino acid one or more of the amino acids at these divergent positions. The positions in bold print are those which in which the murine OB protein as well as the rat OB protein are divergent from the human OB protein, and thus, are particularly suitable for alteration. At one or more of a positions, one may substitute an amino acid from the corresponding rat OB protein, or another amino acid.

The positions from both rat and murine OB protein which diverge from the mature human OB protein are: 4, 32, 33, 35, 50, 64, 68, 71, 74, 77, 78, 89, 97, 100, 102, 105, 106, 107, 108, 111, 118, 136, 138, 142, and 145. An OB protein according to SEQ. ID. NO. 4 having one or more of the above amino acids replaced with another amino acid, such as the amino acid found in the corresponding rat or murine sequence, may also be effective.

Other analogs may be prepared by deleting a part of the protein amino acid sequence. For example, the mature protein lacks a leader sequence (-22 to -1). One may prepare the following truncated forms of human OB protein molecules (using the numbering of SEQ. ID. NO. 4):
(a) amino acids 98-146
(b) amino acids 1-32
(c) amino acids 40-116
(d) amino acids 1-99 and (connected to) 112-146
(e) amino acids 1-99 and (connected to) 112-146 having one or more of amino acids 100-111 placed between amino acids 99 and 112.

In addition, the truncated forms may also have altered one or more of the amino acids which are divergent (in the rat or murine human OB protein) from human OB protein.

The protein (herein the term "protein" is used to include "peptide" and OB analogs, such as those recited infra, unless otherwise indicated) may also be derivatized by the attachment of one or more chemical moieties to the protein moiety. The chemically modified derivatives may be further formulated for intraarterial, intraperitoneal, intramuscular subcutaneous, intravenous, oral, nasal, pulmonary, topical or other routes of administration. Chemical modification of biologically active proteins has been found to provide additional advantages under certain circumstances, such as increasing the stability and circulation time of the therapeutic protein and decreasing immunogenicity. See U.S. Patent No. 4,179,337, Davis et al., issued December 18, 1979. For a review, see Abuchowski et al., in Enzymes as Drugs. (J.S. Holcerberg and J. Roberts, eds. pp. 367-383 (1981)). A review article describing protein modification and fusion proteins is Francis, Focus on Growth Factors 3: 4-10 (May 1992) (published by Mediscript, Mountview Court, Friern Barnet Lane, London N20, OLD, UK).

The chemical moieties suitable for derivatization may be selected from among various water soluble polymers. The polymer selected should be water soluble so that the protein to which it is attached does not precipitate in an aqueous environment, such as a physiological environment. Preferably, for therapeutic use of the end-product preparation, the polymer will be pharmaceutically acceptable. One skilled in the art will be able to select the desired polymer based on such considerations as whether the polymer/protein conjugate will be used therapeutically, and if so, the desired dosage, circulation time, resistance to proteolysis, and other considerations. For the present proteins and peptides, the effectiveness of the derivatization may be ascertained by administering the derivative, in the desired form (i.e., by osmotic pump, or, more preferably, by injection or infusion, or, further formulated for oral, pulmonary or nasal delivery, for example), and observing biological effects.

The water soluble polymer may be selected from the group consisting of, for example, polyethylene glycol, copolymers of ethylene glycol/propylene glycol, carboxymethylcellulose, dextran, polyvinyl alcohol, polyvinyl pyrolidone, poly-1, 3-dioxolane, poly-1,3,6-trioxane, ethylene/maleic anhydride copolymer, polyaminoacids (either homopolymers or random copolymers), and dextran or poly(n-vinyl pyrolidone)polyethylene glycol, propylene glycol homopolymers, polypropylene oxide/ethylene oxide co-polymers, polyoxyethylated polyols and polyvinyl alcohol. Polyethylene glycol propionaldenhyde may have advantages in manufacturing due to its stability in water.

The polymer may be of any molecular weight, and may be branched or unbranched. For polyethylene glycol, the preferred molecular weight is between about 2 kDa and about 100 kDa (the term "about" indicating that in preparations of polyethylene glycol, some molecules will weigh more, some less, than the stated molecular weight) for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a therapeutic protein or analog).

The number of polymer molecules so attached may vary, and one skilled in the art will be able to ascertain the effect on function. One may mono-derivatize, or may provide for a di-, tri-, tetra- or some combination of derivatization, with the same or different chemical moieties (e.g., polymers, such as different weights of polyethylene glycols). The proportion of polymer molecules to protein (or peptide) molecules will vary, as will their concentrations in the reaction mixture. In general, the optimum ratio (in terms of efficiency of reaction in that there is no excess unreacted protein or polymer) will be determined by factors such as the desired degree of derivatization (e.g., mono, di-, tri-, etc.), the molecular weight of the polymer selected, whether the polymer is branched or unbranched, and the reaction conditions.

The polyethylene glycol molecules (or other chemical moieties) should be attached to the protein with consideration of effects on functional or antigenic domains of the protein. There are a number of attachment methods available to those skilled in the art. E.g., EP 0 401 384 herein incorporated by reference (coupling PEG to G-CSF), see also Malik et al., Exp.. Hematol. 20: 1028-1035 (1992) (reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residue. Those having a free carboxyl group may include aspartic acid residues, glutamic acid residues, and the C-terminal amino acid residue. Sulfhydrl groups may also be used as a reactive group for attaching the polyethylene glycol molecule(s). Preferred for therapeutic purposes is attachment at an amino group, such as attachment at the N-terminus or lysine group. Attachment at residues important for receptor binding should be avoided if receptor binding is desired.

One may specifically desire N-terminally chemically modified protein. Using polyethylene glycol as an illustration of the present compositions, one may select from a variety of polyethylene glycol molecules (by molecular weight, branching, etc.), the proportion of polyethylene glycol molecules to protein (or peptide) molecules in the reaction mix, the type of pegylation reaction to be performed, and the method of obtaining the selected N-terminally pegylated protein. The method of obtaining the N-terminally pegylated preparation (i.e., separating this moiety from other monopegylated moieties if necessary) may be by purification of the N-terminally pegylated material from a population of pegylated protein molecules. Selective N-terminal chemical modification may be accomplished by reductive alkylation which exploits differential reactivity of different types of primary amino groups (lysine versus the N-terminal) available for derivatization in a particular protein. Under the appropriate reaction conditions, substantially selective derivatization of the protein at the N-terminus with a carbonyl group containing polymer is achieved. For example, one may selectively N-terminally pegylate the protein by performing the reaction at a pH which allows one to take advantage of the pKₐ differences between the ε-amino group of the lysine residues and that of the α-amino group of the N-terminal residue of the protein. By such selective derivatization, attachment of a water soluble polymer to a protein is controlled: the conjugation with the polymer takes place predominantly at the N-terminus of the protein and no significant modification of other reactive groups, such as the lysine side chain amino groups, occurs. Using reductive alkylation, the water soluble polymer may be of the type described above, and should have a single reactive aldehyde for coupling to the protein. Polyethylene glycol propionaldehyde, containing a single reactive aldehyde, may be used.

An N-terminally monopegylated derivative is preferred for ease in production of a therapeutic. N-terminal pegylation ensures a homogenous product as characterization of the product is simplified relative to di-, tri- or other multi pegylated products. The use of the above reductive alkylation process for preparation of an N-terminal product is preferred for ease in commercial manufacturing. N-terminally monopegylated human recombinant methionyl OB protein 1-146, using a polyethylene glycol of between about 6 kD and about 50 kD is particularly preferred if sustained circulating time of the protein is desired.

The medicaments prepared by the manufacture herein disclosed are pharmaceutical compositions of the proteins and derivatives. Such pharmaceutical compositions may be for administration for injection, or for oral, pulmonary, nasal, transdermal or other forms of administration. In general, comprehended are pharmaceutical compositions comprising effective amounts of protein or derivative products of the invention together with pharmaceutically acceptable diluents, preservatives, solubilizers, emulsifiers, adjuvants and/or carriers. Such compositions include diluents of various buffer content (e.g., Tris-HCl, acetate, phosphate), pH and ionic strength; additives such as detergents and solubilizing agents (e.g., Tween 80, Polysorbate 80), anti-oxidants (e.g., ascorbic acid, sodium metabisulfite), preservatives (e.g., Thimersol, benzyl alcohol) and bulking substances (e.g., lactose, mannitol); incorporation of the material into particulate preparations of polymeric compounds such as polylactic acid, polyglycolic acid, etc. or into liposomes. Hylauronic acid may also be used, and this may have the effect of promoting sustained duration in the circulation. Such compositions may influence the physical state, stability, rate of in vivo release, and rate of in vivo clearance of the present proteins and derivatives. See, e.g., Remington's Pharmaceutical Sciences, 18th Ed. (1990, Mack Publishing Co., Easton, PA 18042) pages 1435-1712 which are herein incorporated by reference. The compositions may be prepared in liquid form, or may be in dried powder, such as lyophilized form. Implantable sustained release formulations are also contemplated, as are transdermal formulations.

Contemplated herein are oral solid dosage forms, which are described generally in Remington's Pharmaceutical Sciences, 18th Ed. 1990 (Mack Publishing Co. Easton PA 18042) at Chapter 89, which is herein incorporated by reference. Solid dosage forms include tablets, capsules, pills, troches or lozenges, cachets or pellets. Also, liposomal or proteinoid encapsulation may be used to formulate the present compositions (as, for example, proteinoid microspheres reported in U.S. Patent No. 4,925,673). Liposomal encapsulation may be used and the liposomes may be derivatized with various polymers (E.g., U.S. Patent No. 5,013,556). A description of possible solid dosage forms for the therapeutic is given by Marshall, K. In: *Modem Pharmaceutics* Edited by G.S. Banker and C.T. Rhodes Chapter 10, 1979, herein incorporated by reference. In general, the formulation will include the protein (or analog or derivative), and inert ingredients which allow for protection against the stomach environment, and release of the biologically active material in the intestine.

Also specifically contemplated are oral dosage forms of the above derivatized proteins. Protein may be chemically modified so that oral delivery of the derivative is efficacious. Generally, the chemical modification contemplated is the attachment of at least one moiety to the protein (or peptide) molecule itself, where said moiety permits (a) inhibition of proteolysis; and (b) uptake into the blood stream from the stomach or intestine. Also desired is the increase in overall stability of the protein and increase in circulation time in the body. Examples of such moieties include: Polyethylene glycol, copolymers of ethylene glycol and propylene glycol, carboxymethyl cellulose, dextran, polyvinyl alcohol, polyvinyl pyrrolidone and polyproline. Abuchowski and Davis, Soluble Polymer-Enzyme Adducts. In: "Enzymes as Drugs", Hocenberg and Roberts, eds., Wiley-Interscience, New York, NY, (1981), pp 367-383; Newmark, et al., J. Appl. Biochem. 4: 185-189 (1982). Other polymers that could be used are poly-1,3-dioxolane and poly-1,3, 6-tioxocane. Preferred for pharmaceutical usage, as indicated above, are polyethylene glycol moieties.

For the protein (or derivative) the location of release may be the. stomach, the small intestine (the duodenum, the jejunem, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the protein (or derivative) or by release of the biologically active material beyond the stomach environment, such as in the intestine.

To ensure full gastric resistance a coating impermeable to at least pH 5.0 is essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl- acetate phthalate (PVAP), Eudragit L30D, Aquateric, cellulose acetate phthalate (CAP), Eudragit L, Eudragit S, and Shellac. These coatings may be used as mixed films.

A coating or mixture of coatings can also be used on tablets, which are not intended for protection against the stomach. This can include sugar coatings, or coatings which make the tablet easier to swallow. Capsules may consist of a hard shell (such as gelatin) for delivery of dry therapeutic i.e. powder; for liquid forms, a soft gelatin shell may be used. The shell material of cachets could be thick starch or other edible paper. For pills, lozenges, molded tablets or tablet triturates, moist massing techniques can be used.

The therapeutic can be included in the formulation as fine multiparticulates in the form of granules or pellets of particle size about 1mm. The formulation of the material for capsule administration could also be as a powder, lightly compressed plugs or even as tablets. The therapeutic could be prepared by compression.

Colorants and flavoring agents may all be included. For example, the protein (or derivative) may be formulated (such as by liposome or microsphere encapsulation) and then further contained within an edible product, such as a refrigerated beverage containing colorants and flavoring agents.

One may dilute or increase the volume of the therapeutic with an inert material. These diluents could include carbohydrates, especially mannitol, α-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the therapeutic into a solid dosage form. Materials used as disintegrates include but are not limited to starch including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold the therapeutic agent together to form a hard tablet and include materials from natural products such as acacia, tragacanth, starch and.gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An antifrictional agent may be included in the formulation of the therapeutic to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of the therapeutic into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. The list of potential nonionic detergents that could be included in the formulation as surfactants are lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation of the protein or derivative either alone or as a mixture in different ratios.

Additives which potentially enhance uptake of the protein (or derivative) are for instance the fatty acids oleic acid, linoleic acid and linolenic acid.

Controlled release formulation may be desirable. The drug could be incorporated into an inert matrix which permits release by either diffusion or leaching mechanisms i.e. gums. Slowly degenerating matrices may also be incorporated into the formulation. Another form of a controlled release of this therapeutic is by a method based on the Oros therapeutic system (Alza Corp.), i.e. the drug is enclosed in a semipermeable membrane which allows water to enter and push drug out through a single small opening due to osmotic effects. Some entric coatings also have a delayed release effect.

Other coatings may be used for the formulation. These include a variety of sugars which could be applied in a coating pan. The therapeutic agent could also be given in a film coated tablet and the materials used in this instance are divided into 2 groups. The first are the nonenteric materials and include methyl cellulose, ethyl cellulose, hydroxyethyl cellulose, methylhydroxy-ethyl cellulose, hydroxypropyl cellulose, hydroxypropyl-methyl cellulose, sodium carboxy-methyl cellulose, providone and the polyethylene glycols. The second group consists of the enteric materials that are commonly esters of phthalic acid.

A mix of materials might be used to provide the optimum film coating. Film coating may be carried out in a pan coater or in a fluidized bed or by compression coating.

Also contemplated is pulmonary delivery of the present protein (or derivatives thereof). The protein (or derivative) would be delivered to the lungs of a mammal while inhaling and traverses across the lung epithelial lining to the blood stream. (Other reports of this include Adjei et al., Pharmaceutical Research 7: 565-569 (1990); Adjei et al., International Journal of Pharmaceutics 63: 135-144. (1990)(leuprolide acetate); Braquet et al., Journal of Cardiovascular Pharmacology 13(suppl. 5): s.143-146 (1989)(endothelin-1);Hubbard et al., Annals of Internal Medicine 3: 206-212 (1989)(α1-antitrypsin); Smith et al., J. Clin. Invest. 84: 1145-1146 (1989)(α-1-proteinase); Oswein et al., "Aerosolization of Proteins", Proceedings of Symposium on Respiratory Drug Delivery II, Keystone, Colorado, March, 1990 (recombinant human growth hormone); Debs et al., The Journal of Immunology 140: 3482-3488 (1988)(interferon-γ and tumor necrosis factor alpha) and Platz et al., U.S. Patent No. 5,284,656 (granulocyte colony stimulating factor).

Contemplated for use in the practice of this invention are a wide range of mechanical devices designed for pulmonary delivery of therapeutic products, including but not limited to nebulizers, metered dose inhalers, and powder inhalers, all of which are familiar to those skilled in the art.

Some specific examples of commercially available devices suitable for the practice of this invention are the Ultravent nebulizer, manufactured by Mallinckrodt, Inc., St. Louis, Missouri; the Acorn II nebulizer, manufactured by Marquest Medical Products, Englewood, Colorado; the Ventolin metered dose inhaler, manufactured by Glaxo Inc., Research Triangle Park, North Carolina; and the Spinhaler powder inhaler, manufactured by Fisons Corp., Bedford, Massachusetts.

All such devices require the use of formulations suitable for the dispensing of protein (or analog or derivative). Typically, each formulation is specific to the type of device employed and may involve the use of an appropriate propellant material, in addition to diluents, adjuvants and/or carriers useful in therapy.

The protein (or derivative) should most advantageously be prepared in particulate form with an average particle size of less than 10 µm (or microns), most preferably 0.5 to 5 µm, for most effective delivery to the distal lung.

Carriers include carbohydrates such as trehalose, mannitol, xylitol, sucrose, lactose, and sorbitol. Other ingredients for use in formulations may include DPPC, DOPE, DSPC and DOPC. Natural or synthetic surfactants may be used. Polyethylene glycol may be used (even apart from its use in derivatizing the protein or analog). Dextrans, such as cyclodextran, may be used. Bile salts and other related enhancers may be used. Cellulose and cellulose derivatives may be used. Amino acids may be used, such as use in a buffer formulation.

Also, the use of liposomes, microcapsules or microspheres, inclusion complexes, or other types of carriers is contemplated.

Formulations suitable for use with a nebulizer, either jet or ultrasonic, will typically comprise protein (or derivative) dissolved in water at a concentration of about 0.1 to 25 mg of biologically active protein per mL of solution. The formulation may also include a buffer and a simple sugar (e.g., for protein stabilization and regulation of osmotic pressure). The nebulizer formulation may also contain a surfactant, to reduce or prevent surface induced aggregation of the protein caused by atomization of the solution in forming the aerosol.

Formulations for use with a metered-dose inhaler device will generally comprise a finely divided powder containing the protein (or derivative) suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Formulations for dispensing from a powder inhaler device will comprise a finely divided dry powder containing protein (or derivative) and may also include a bulking agent, such as lactose, sorbitol, sucrose, mannitol, trehalose, or xylitol in amounts which facilitate dispersal of the powder from the device, e.g., 50 to 90% by weight of the formulation.

Nasal delivery of the protein (or analog or derivative) is also contemplated. Nasal delivery allows the passage of the protein to the blood stream directly after administering the therapeutic product to the nose, without the necessity for deposition of the product in the lung. Formulations for nasal delivery include those with dextran or cyclodextran. Delivery via transport across other mucus membranes is also contemplated.

One skilled in the art will be able to ascertain effective dosages by administration and observing the desired therapeutic effect. Preferably, the formulation of the molecule will be such that between about .10 µg/kg/day and 10 mg/kg/day will yield the desired therapeutic effect. The effective dosages may be determined using diagnostic tools over time. For example, a diagnostic for measuring the amount of OB protein in the blood (or plasma or serum) may first be used to determine endogenous levels of OB protein. Such diagnostic tool may be in the form of an antibody assay, such as an antibody sandwich assay. The amount of endogenous OB protein is quantified initially, and a baseline is determined. The therapeutic dosages are determined as the quantification of endogenous and exogenous OB protein (that is, protein, analog or derivative found within the body, either-self-produced or administered) is continued over the course of therapy. The dosages may therefore vary over the course of therapy, with a relatively high dosage being used initially, until therapeutic benefit is seen, and lower dosages used to maintain the therapeutic benefits.

Generally, effective dosages for humans desiring a reduction in blood lipid levels will be sufficient to reduce or maintain reduced cholesterol or triglyceride levels in a normal range. Persons with blood cholesterol levels above about 200 milligrams per 100 milliliters will benefit from reduced cholesterol levels. Persons with triglyceride levels above about 250 milligrams per 100 milliliters will generally benefit from reduction or maintenance of a lowered triglyceride level.

Ideally, in situations where solely reduction in blood lipid levels is desired, or where maintenance of reduction of blood lipid levels is desired, the dosage will be insufficient to result in weight loss. Thus, during an initial course of therapy of an obese person, dosages may be administered whereby weight loss and concomitant blood lipid level lowering is achieved. Once sufficient weight loss is achieved, a dosage sufficient to prevent re-gaining weight, yet sufficient to maintain desired blood lipid levels may be administered. These dosages can be determined empirically, as the effects of OB protein are reversible. E.g., Campfield et al., Science 269: 546-549 (1995) at 547. Thus, if a dosage resulting in weight loss is observed when weight loss is not desired, one would administer a lower dose in order to achieve the desired blood lipid levels, yet maintain the desired weight.

The present invention may be used in conjunction with other medicaments, such as those useful for the treatment of diabetes (e.g., insulin, and possibly amylin), cholesterol and blood pressure lowering medicaments (such as those recited above), and activity increasing medicaments (e.g., amphetamines). Appetite suppressants may also be used. Such administration may be simultaneous or may be in seriatim.

In addition, the present invention may be used in conjunction with surgical procedures, such as cosmetic surgeries designed to alter the overall appearance of a body (e.g., liposuction or laser surgeries designed to reduce body mass). The health benefits of cardiac surgeries, such as bypass surgeries or other surgeries designed to relieve a deleterious condition caused by blockage of blood vessels by fatty deposits, such as arterial plaque, may be increased with concomitant use of the present compositions and methods. Methods to eliminate gall stones, such as ultrasonic or laser methods, may also be used either prior to, during or after a course of the present therapeutic use.

Therefore, the present invention provides a means for reducing the level of blood lipids in a non-obese patient, or maintaining a reduced level of blood lipids in a patient having an elevated level of blood lipids, comprised of the administration of an amount of an OB protein, analog or derivative thereof sufficient to reduce the blood lipid level or maintain said reduced level but insufficient to result in weight loss, said OB protein, analog or derivative thereof selected from:
(a) the amino acid sequence 1-146 as set forth in SEQ. ID. NO. 2 (below) or SEQ ID. NO. 4 (below),
(b) the amino acid sequence set 1-146 as forth in SEQ. ID. NO. 4 (below) having a lysine residue at position 35 and an isoleucine residue at position 74;
(c) the amino acid sequence of subpart (b) having a different amino acid substituted in one or more of the following positions (using the numbering according to SEQ. ID. NO. 4 and retaining the same numbering even in the absence of a glutaminyl residue at position 28): 4, 32, 33, 35, 50, 64, 68, 71, 74, 77, 78, 89, 97, 100, 102, 105, 106, 107, 108, 111, 118, 136, 138, 142, and 145;
(d) the amino acid sequence of subparts (a), (b) or (c) optionally lacking a glutaminyl residue at position 28;
(e) the amino acid sequence of subparts (a), (b), (c), or (d) having a methionyl residue at the N terminus.
(f) a truncated OB protein analog selected from among: (using the numbering of SEQ. ID. NO. 4):
   (i) amino acids 98-146
   (ii) amino acids 1-32
   (iii) amino acids 40-116
   (iv) amino acids 1-99 and 112-146
   (v) amino acids 1-99 and 112-146 having one or more of amino acids 100-111 placed between amino acids 99 and 112; and,
   (vi) the truncated OB analog of subpart (i) having one or more of amino acids 100, 102, 105, 106, 107, 108, 111, 118, 136, 138, 142, and 145 substituted with another amino acid;
   (vii) the truncated analog of subpart (ii) having one or more of amino acids 4 and 32 substituted with another amino acid;
   (viii) the truncated analog of subpart (iii) having one or more of amino acids 50, 64, 68, 71, 74, 77, 78, 89, 97, 100, 102, 105, 106, 107, 108, and 111 replaced with another amino acid;
   (vix) the truncated analog of subpart (iv) having one or more of amino acids 4, 32, 33, 35, 50, 64, 68, 71, 74, 77, 78, 89, 97, 118, 136, 138, 142, and 145 replaced with another amino acid;
   (x) the truncated analog of subpart (v) having one or more of amino acids 4, 32, 33, 35, 50, 64, 68, 71, 74, 77, 78, 89, 97, 100, 102, 105, 106, 107, 108, 111, 118, 136, 138, 142, and 145 replaced with another amino acid;
   (xi) the truncated analog of any of subparts (i)-(x) having an N-terminal methionyl residue; and
(g) the OB protein or analog derivative of any of subparts (a) through (f) comprised of a chemical moiety connected to the protein moiety;
(h) a derivative of subpart (g) wherein said chemical moiety is a water soluble polymer moiety;
(i) a derivative of subpart (h) wherein said water soluble polymer moiety is polyethylene glycol; and
(j) a derivative of subpart (i) wherein said polyethylene glycol moiety is attached at solely the N-terminus of said protein moiety
(h) an OB protein, analog or derivative of any of subparts (a) through (h) in a pharmaceutically acceptable carrier.

In another aspect, the present invention provides a means as above wherein said non obese patient has an elevated level of serum cholesterol, or has had such elevated level, and the OB protein analog or derivative dosage is sufficient to reduce the level and/or maintain the serum cholesterol level of said patient at normal levels. Preferably this level will be at or below 240 mg cholesterol/100 ml, and more preferably, at or below 200 mg cholesterol/100 ml.

In another aspect, the present invention provides a means as above wherein the non-obese patient has an elevated level of serum triglycerides, and the OB protein analog or derivative dosage is sufficient to reduce the triglyceride level and/or maintain the triglyceride level of said patient at normal levels. Preferably this level will be at or below 500 mg/100 ml and more preferably at or below 250 mg/100 ml. Particularly, an individual with, for instance, familial hypertriglyceridemia, may have elevated triglyceride levels yet normal cholesterol levels, and may benefit from the present invention.

In yet another aspect, the present invention provides a means as above wherein the non-obese patient has an elevated level of arterial plaque, and the OB protein analog or derivative dosage is sufficient to reduce the level of arterial plaque or maintain the arterial plaque level of said patient at normal levels. Preferably the dosage will be sufficient to allow or maintain normal blood flow through the affected blood vessels.

Furthermore, as the formation of gall stones in the gall bladder is related to the blood cholesterol level, the present invention provides a means as above to prevent or reduce the formation of gall stones.

The present invention provides for use or the above OB protein, analogs and derivatives thereof for manufacture of a medicament for use in the treatment of elevated levels of blood lipids, including the treatment of high cholesterol, elevated levels of triglycerides, hypertension and high levels of arterial plaque, and in the prevention or reduction in the formation of gall stones as described above.

The following examples are offered to more fully illustrate the invention, but are not to be construed as limiting the scope thereof.

### EXAMPLE 1

This example demonstrates that in normal mice which are not obese and do not have elevated blood lipid levels, administration of murine recombinant OB protein results in a lowering of cholesterol and triglyceride levels. Normal CD1 mice were administered recombinant murine OB protein via continuous infusion (see Materials and Methods, below). At the dosages administered, the animals lost weight. As shown in Table 1, the mice had substantial reduction of serum cholesterol and triglycerides in a dose-dependent fashion:

**TABLE 1**

| Dose | Cholesterol | Triglycerides |
|---|---|---|
| PBS (8 mice) | 103.5 +/-7.4 | 81.625 +/- 9.0 |
| 0.03 mg/kg/day (7 mice) | 95.0 +/- 6.54 | 86.143 +/- 3.7 |
| 0.1 mg/kq/day (9 mice) | 73.11 +/- 5.3 | 67.0 +/- 9.4 |
| 0.3 mg/kg/day (8 mice) | 76.88 +/- 9.0 | 55.38 +/- 7.5 |
| 1.0 mq/kq/day (8 mice) | 66 +/- 7.9 | 38.9 +/-3.8 |

Similar results were also seen for ob/ob mutant mice, which also were observed to lose weight (Table 2, below), although C57 (+/+) having normal levels of blood lipids showed no change:

**TABLE 2**

| Group/dose | Cholesterol | Triqlycerides |
|---|---|---|
| C57(+/+) 0 | 65.5 +/- 2.67 | 55 +/- 3.46 |
| C57 (+/+) 0.1 mg/kg/day | 67.8 +/- 2.37 | 57.4 +/- 4.4 |
| C57 (+/+) 1 mq/kq/day | 79.8 +/- 9.15 | 73.6 +/- 8.15 |
| C57 (+/+) 10 mq/kq/day | 71.8 +/- 9.44 | 45.8 +/- 5.23 |
| ob/ob 0 | 122.7 +/- 8.5 | 122.2 +/- 19.7 |
| ob/ob 0.1 mg/kg/day | 117.6 +/- 3.43 | 110 +/- 7.38 |
| ob/ob 1 mg/kg/day | 82.4 +/- 4.5 | 78.6 +/- 3.3 |
| ob/ob 10 mg/kg/day | 73.55 +/- 3.9 | 67.33 +/- 11.3 |

These data demonstrate that the OB protein, or analogs or derivatives thereof, are effective blood lipid lowering agents.

### EXAMPLE 2

A non-obese human patient undergoes coronary bypass surgery or other invasive treatment to alleviate advanced stages arterial plaque formation. After the surgery, the patient is administered a maintenance dose of OB protein or analog or derivative in order to prevent the re-formation of arterial plaque. The dose administered is insufficient to result in weight loss. Administration is chronic. Levels of circulating OB protein or analog or derivative may be monitored using a diagnostic kit, such as an antibody assay against the OB protein (or other antigenic source if applicable).

### EXAMPLE 3

A non-obese human patient experiences hypertension due to restricted blood flow from clogged arteries. The patient is administered a dose of OB protein, or analog or derivative thereof sufficient to reduce arterial plaque resulting in clogged arteries. Thereafter, the patient is monitored for further arterial plaque formation, and hypertension. If the condition re-appears, the patient is re-administered an effective amount of OB protein, analog or derivative sufficient to restore blood flow, yet insufficient to result in weight loss. Levels of circulating OB protein or analog or derivative may be monitored using a diagnostic kit, such as an antibody assay against the OB protein (or other antigenic source if applicable).

### EXAMPLE 4

A human patient experiences gall stones. Either the gall stones are not removed and the formation of additional gall stones is sought to be avoided, or the gall stones are removed but the gall bladder remains (as, for example, using laser or ultrasonic surgery) and the formation of additional gall stones is sought to be avoided. The patient is administered an effective amount of OB protein, analog or derivative thereof to result in prevention of accumulation of additional gall stones or re-accumulation of gall stones. Levels of circulating OB protein or analog or derivative may be monitored using a diagnostic kit, such as an antibody assay against the OB protein (or other antigenic source if applicable).

### MATERIALS AND METHODS

Animals: Wild type CD1 mice were used for Example 1 (Table 1 data). Animals were maintained under humane conditions. Also, C57 +/+ or ob/ob mice were used for Example 1 (Table 2 data).

Administration of Protein or Vehicle. For Example 1, (Table 1 data) recombinant murine protein (as described below) or vehicle (phosphate buffered saline, "PBS", pH 7.4) was administered by osmotic pump infusion. Alzet osmotic minipumps (Alza, Palo Alto, CA, model no. 1007D) were surgically placed in each mice in a subcutaneous pocket in the subscapular area. The pumps were calibrated to administer 0.5 µl protein in solution per hour for the dosages indicated in Table 1. In the study resulting in the data of Table 2, mice were injected once daily with the listed dosages of recombinant murine OB protein as set forth below.

Protein: Sequence ID Nos. 1 and 2 set forth murine recombinant OB DNA and protein, and Sequence ID Nos. 3 and 4 set forth recombinant human OB analog DNA and protein. Murine recombinant protein as in SEQ. ID NO. 2 was used in EXAMPLE 1. As indicated above, these are illustrative of the OB protein which may be used in the present methods of treatment and manufacture of a medicament. Other OB proteins or analogs or derivatives thereof may be used.

Herein, the first amino acid of the amino acid sequence for recombinant protein is referred to as +1, and is valine, and the amino acid at position -1 is methionine. The C-terminal amino acid is number 146 (cysteine).

### Recombinant murine met OB (double stranded) DNA and amino acid sequence (Seq. ID. Nos. 1 and 2):

### Recombinant human met OB analog (Double Stranded) DNA and amino acid sequence (SEQ. ID. Nos. 3 and 4)

### METHODS FOR PRODUCTION

The following methods were used to produce the recombinant murine protein (SEQ. ID. NO. 2) as used in Example 1.

### Expression Vector and Host Strain

The plasmid expression vector used is pCFM1656, ATCC Accession No. 69576. The above DNA was be ligated into the expression vector pCFM1656 linearized with XbaI and BamHI and transformed into the E. coli host strain, FM5. E. coli FM5 cells were derived at Amgen Inc., Thousand Oaks, CA from E. coli K-12 strain (Bachmann, et al., Bacteriol. Rev. 40: 116-167 (1976)) and contain the integrated lambda phage repressor gene, cI₈₅₇ (Sussman et al., C.R. Acad. Sci. 254: 1517-1579 (1962)). Vector production, cell transformation, and colony selection were performed by standard methods. E.g., Sambrook, et al., Molecular Cloning: A Laboratory Manual, 2d Edition, Cold Spring Harbor Laboratory Press, Cold Spring Harbor, NY. Host cells were be grown in LB media.

Fermentation Process A three-phase fermentation protocol known as a fed-batch process was used. Media compositions are set forth below.

Batch: A nitrogen and phosphate source were sterilized (by raising to 122 °C for 35 minutes, 18-20 psi) in the fermentation vessel (Biolafitte, 12 liter capacity). Upon cooling, carbon, magnesium, vitamin, and trace metal sources were added aseptically. An overnight culture of the above recombinant murine protein-producing bacteria (16 hours or more) of 500 mL (grown in LB broth) was added to the fermentor.

Feed I: Upon reaching between 4.0-6.0 OD₆₀₀, cultures were fed with Feed I. The glucose was fed at a limiting rate in order to control the growth rate (µ). An automated system (called the Distributive Control System) was instructed to control the growth rate to 0.15 generations per hour.

Feed II: When the OD₆₀₀ had reached 30, culture temperature were slowly increased to 42°C and the feed changed to Feed II, below. The fermentation was allowed to continue for 10 hours with sampling every 2 hours. After 10 hours, the contents of the fermentor was chilled to below 20°C and harvested by centrifugation.

| Media Composition: | | |
|---|---|---|
| Batch | 10 g/L | Yeast extract |
| | 5.25 g/L | (NH₄)₂SO₄ |
| | 3.5 g/L | K₂HPO₄ |
| | 4.0 g/L | KH₂PO₄ |
| | 5.0 g/L | Glucose |
| | 1.0 g/L | MgSO₄·7H₂O |
| | 2.0 mL/L | Vitamin Solution |
| | 2.0 mL/L | Trace Metal Solution |
| | 1.0 mL/L | P2000 Antifoam |
| Feed I | 50 g/L | Bacto-tryptone |
| | 50 g/L | Yeast extract |
| | 450 g/L | Glucose |
| | 8.75 g/L | MgSO₄·7H₂O |
| | 10 mL/L | Vitamin Solution |
| | 10 mL/L | Trace Metal Solution |
| Feed II | 200 g/L | Bacto-tryptone |
| | 100 g/L | Yeast extract |
| | 110 g/L | Glucose |

Vitamin Solution (Batch and Feed I): 0.5 g Biotin, 0.4 g Folic acid, and 4.2 g riboflavin, was dissolved in 450 mls H₂O and 3 mls 10 N NaOH, and brought to 500 mLs in H₂O. 14 g pyridoxine-HCl and 61 g niacin was dissolved 150 ml H₂O and 50 ml 10 N NaOH, and brought to 250 ml in H₂O. 54 g pantothenic acid was dissolved in 200 mL H₂O, and brought to 250 mL. The three solutions were combined and brought to 10 liters total volume.

Trace Metal Solution (Batch and Feed I):
Ferric Chloride (FeCl₃·6H₂O): 27 g/L
Zinc Chloride (ZnCl₂·4H₂O): 2 g/L
Cobalt Chloride (CoCl₂·6H₂O): 2 g/L
Sodium Molybdate (NaMoO₄-2H₂O): 2 g/L
Calcium Chloride (CaCl₂·2H₂O): 1 g/L
Cupric Sulfate (CuSO₄·5H₂O): 1.9 g/L
Boric Acid (H₃BO₃): 0.5 g/L
Manganese Chloride (MnCl₂·4H₂O): 1.6 g/L
Sodium Citrate dihydrate: 73.5 g/L

### Purification Process for Murine OB Protein

Purification was accomplished by the following steps (unless otherwise noted, the following steps were performed at 4°C):
1. Cell paste. E. coli cell paste was suspended in 5 times volume of 7 mM of EDTA, pH 7.0. The cells in the EDTA were further broken by two passes through a microfluidizer. The broken cells were centrifuged at 4.2 K rpm for 1 hour in a Beckman J6-B centrifuge with a JS-4.2 rotor.
2. Inclusion body wash #1. The supernatant from above was removed, and the pellet was resuspended with 5 times volume of 7 mM EDTA, pH 7.0, and homogenized. This mixture was centrifuged as in step 1.
3. Inclusion body wash #2. The supernatant from above was removed, and the pellet was resuspended in ten times volume of 20 mM tris, pH 8.5, 10 mM DTT, and 1% deoxycholate, and homogenized. This mixture was centrifuged as in step 1.
4. Inclusion body wash #3. The supernatant from above was removed and the pellet was resuspended in ten times volume of distilled water, and homogenized. This mixture was centrifuged as in step 1.
5. Refolding. The pellet was refolded with 15 volumes of 10 mM HEPES, pH 8.5, 1% sodium sarcosine (N-lauroyl sarcosine), at room temperature. After 60 minutes, the solution was made to be 60 µM copper sulfate, and then stirred overnight.
6. Removal of sarcosine. The refolding mixture was diluted with 5 volumes of 10 mM.tris buffer, pH 7.5, and centrifuged as in step 1. The supernatant was collected, and mixed with agitation for one hour with Dowex® 1-X4 resin (Dow Chemical Co., Midland MI), 20-50 mesh, chloride form, at 0.066% total volume of diluted refolding mix. See WO 89/10932 at page 26 for more information on Dowex®. This mixture was poured into a column and the eluant collected. Removal of sarcosine was ascertained by reverse phase HPLC.
7. Acid precipitation. The eluant from the previous step was collected, and pH adjusted to pH 5.5, and incubated for 30 minutes at room temperature. This mixture was centrifuged as in step 1.
8. Cation exchange chromatography. The pH of the supernatant from the previous step was adjusted to pH 4.2, and loaded on CM Sepharose Fast Flow (at 7% volume). 20 column volumes of salt gradient were done at 20 mM NaOAC, pH 4.2, 0 M to 1.0 M NaCl.
9. Hydrophobic interaction chromatography. The CM Sepharose pool of peak fractions (ascertained from ultraviolet absorbance) from the above step was made to be 0.2 M ammonium sulfate. A 20 column volume reverse salt gradient was done at 5 mM NaOAC, pH 4.2, with .4 M to 0 M ammonium sulfate. This material was concentrated and diafiltered into PBS.

### Fermentation of recombinant human OB protein

analog: Fermentation of the above host cells to produce recombinant human OB protein analog (SEQ. ID. NO. 4) can be accomplished using the conditions and compositions as described above for recombinant murine material.

### Purification of the recombinant human OB

protein: Recombinant human protein may be purified using methods similar to those used for purification of recombinant murine protein, as in Example 1, above. For preparation of recombinant human OB protein analog, step 8 should be performed by adjusting the pH of the supernatant from step 7 to pH 5.0, and loading this onto a CM Sepharose fast flow column. The 20 column volume salt gradient should be performed at 20 mM NaOAC, pH 5.5, OM to 0.5 M NaCl. Step 9 should be performed by diluting the CM Sepharose pool four fold with water, and adjusting the pH to 7.5. This mixture should be made to 0.7 M ammonium sulfate. Twenty column volume reverse salt gradient should be done at 5 mM NaOAC, pH 5.5, 0.2 M to OM ammonium sulfate. Otherwise, the above steps are identical.

## Claims

1. Use of an OB protein, analog or derivative thereof for the manufacture of a medicament for reducing the level of blood lipids in a non-obese patient, or maintaining a reduced level of blood lipids in a non-obese patient having an elevated level of blood lipids, wherein the OB protein, analog, or derivative thereof is in an amount sufficient to reduce or maintain reduced levels of said blood lipids, but insufficient to cause weight loss, and wherein said OB protein, analog, or derivative thereof is selected from among:
(a) che amino acid sequence 1-146 as sec forth in SEQ. ID. NO 2 or SEQ ID. NO. 4;
(b) the amino acid sequence set 1-146 as forth in SEQ. ID. NO. 4 having a lysine residue at position 35 and an isoleucine residue at position 74;
(c) the amino acid sequence of subpart (b) having a different amino acid substituted in one or more of the following positions (using the numbering according to SEQ. ID. NO. 4, and retaining the same numbering even in the absence of a glutaminyl residue at position 28): 4, 32, 33, 35, 50, 64, 68, 71, 74, 77, 78, 89, 97, 100, 102, 105, 106, 107, 108, 111, 118, 136, 138, 142, and 145;
(d) the amino acid sequence of subparts (a), (b) or (c) optionally lacking a glutaminyl residue at position 28;
(e) the amino acid sequence of subparts (a), (b), (c). or (d) having a methionyl residue at the N terminus;
(f) a truncated OB protein analog selected from among: (using the numbering of. SEQ. ID. NO. 4):
(i) amino acids 98-146
(ii) amino acids 1-32
(iii) amino acids 40-116
(iv) amino acids 1-99 and 112-146
(v) amino acids 1-99 and 112-146 having one or more of amino acids 100-111 placed between amino acids 99 and 112;
(vi) the truncated OB analog of subpart (i) having one or more of amino acids 100, 102, 105, 106, 107, 108, 111, 118, 136, 138, 142, and 145 substituted with another amino acid;
(vii) the truncated analog of subpart (ii) having one or more of amino acids 4 and 32 substituted with another amino acid;
(viii) the truncated analog of subpart (iii) having one or more of amino acids 50, 64, 68, 71, 74, 77, 78, 89, 97, 100, 102, 105, 106, 107, 108, and 111 replaced with another amino acid:
(vix) the truncated analog of subpart (iv) having one or more of amino acids 4, 32, 33, 35, 50, 64, 68, 71, 74, 77, 78, 89, 97, 118, 136, 138, 142, and 145 replaced with another amino acid;
(x) the truncated analog of subpart (v) having one or more of amino acids 4, 32, 33, 35, 50, 64, 68, 71, 74, 77, 78, 89, 97, 100, 102, 105, 106, 107, 108, 111, 118, 136, 138, 142, and 145 replaced with another amino acid;
(xi) the truncated analog of any of subparts (i)-(x) having an N-terminal methionyl residue; and
(g) the OB protein or analog derivative of any of subparts (a) through (f) comprised of a chemical moiety connected to the protein moiety;
(h) a derivative of subpart (g) wherein said chemical moiety is a water soluble polymer moiety;
(i) a derivative of subpart (h) wherein said water soluble polymer moiety is polyethylene glycol;
(j) a derivative of subpart (i) wherein said polyethylene glycol moiety is attached at solely the N-terminus of said protein moiety; and
(h) an OB protein, analog or derivative of any of subparts (a) through (h) in a pharmaceutically acceptable carrier.

2. A use of claim 1 wherein said patient has an elevated level of serum cholesterol, and said OB protein, analog, or derivative dosage is sufficient to maintain the serum cholesterol level of said patient at normal levels.

3. A use of claim 1 wherein said patient has an elevated level of serum triglycerides, and the OB protein, analog, or derivative dosage is sufficient co maintain the triglyceride level of said patient at normal levels.

4. A use of claim 1 wherein said patient has an elevated level of arterial plaque, and the OB protein, analog, or derivative dosage is sufficient co maintain the arterial plaque level of said patient at normal levels.

5. A use of claim 4 wherein reduction of arterial plaque results in treatment of hypertension.

6. A use of claim 1 wherein said patient has currently or has previously had gall stones, and the OB protein, analog, or derivative dosage is sufficient to prevent or reduce the formation of additional gall stones.

## Patentansprüche

1. Verwendung eines OB-Proteins, Analogs oder Derivats davon zur Herstellung eines Medikaments zum Reduzieren der Konzentration von Lipiden im Blut in einem nichtfertleibigen Patienten oder zum Aufrechterhalten einer verringerten Konzentration von Lipiden im Blut in einem einem nicht-fettleibigen Patienten mit einer erhöhten Konzentration von Lipiden im Blut, wobei das OB-Protein, Analog oder Derivat davon in einer Menge vorliegt, die ausreicht, um verringerte Konzentrationen besagter Lipide im Blut zu verringern oder aufrechtzuerhalten, die aber nicht ausreicht, um einen Gewichtsverlust zu verursachen, und wobei besagtes OB-Protein, Analog oder Derivat davon ausgewählt ist aus:
(a) der Aminosäuresequenz 1-146, wie dargestellt in SEQ ID NO: 2 oder SEQ ID NO: 4
(b) der Aminosäuresequenz 1-146, wie dargestellt in SEQ ID NO: 4, mit einem Lysinrest an Position 35 und einem Isoleucinrest an Position 74;
(c) der Aminosäuresequenz des Teils (b) mit einer unterschiedlichen Aminosäure substituiert an einer oder mehreren der folgenden Positionen (unter Verwendung der Numerierung gemäß SEQ ID NO: 4 und unter Beibehaltung derselben Numerierung sogar in der Abwesenheit eines Glutaminrests an Position 28); 4, 32, 33, 35, 50, 64, 68, 71, 74, 77, 78, 89, 97,100, 102, 105, 106, 107,108, 111, 118, 136, 138, 142, und 145;
(d) der Aminosäuresequenz der Teile (a), (b) oder (c), der fakultativ ein Glutaminrest an Position 28 fehlt;
(e) der Aminosäuresequenz der Teile (a), (b), (c), oder (d) mit einem Methionylrest am N-Terminus;
(f) einem trunkierten OB-Proteinanalog, ausgewählt aus: (unter Verwendung der Numerierung von SEQ ID NO: 4):
(i) Aminosäuren 98-146
(ii) Aminosäuren 1-32
(iii) Aminosäuren 40-116
(iv) Aminosäuren 1-99 und 112-146
(v) Aminosäuren 1-99 und 112-146 mit einer oder mehreren der Aminosäuren 100-111 eingebracht zwischen Aminosäuren 99 und 112;
(vi) dem trunkierten OB-Analog des Teils (i) mit einer oder mehreren der Aminosäuren 100, 102, 105, 106, 107,108,111, 118, 136, 138, 142 und 145 substituiert mit einer anderen Aminosäure;
(vii) dem trunkierten Analog des Teils (ii) mit einer oder mehreren der Aminosäuren 4 und 32 substituiert mit einer anderen Aminosäure;
(viii) dem trunkierten Analog des Teils (iii) mit einer oder mehreren der Aminosäuren 50, 64, 68, 71, 74, 77, 78, 89, 97, 100, 102, 10i, 106, 107, 108 und 111 ersetzt durch eine andere Aminosäure;
(vix) dem trunkierten Analog des Teils (iv) mit einer oder mehreren der Aminosäuren 4, 32, 33, 35, 50, 64, 68, 71, 74, 77, 78, 89, 97, 18, 136, 138, 142, und 145 ersetzt durch eine andere Aminosäure;
(x) dem trunkierten Analog des Teils (v) mit einer oder mehreren der Aminosäuren 4, 32, 33, 35, 50, 64, 68, 71, 74, 77, 78, 89, 97, 100, 102, 105, 106, 107, 108, 111, 118, 136, 138, 142, und 145 ersetzt durch eine andere Aminosäure;
(xi) dem trunkierten Analog eines der Teile (i) - (x) mit einem N-terminalen Methionylrest; und
(g) dem OB-Protein oder Analogderivat von einem der Teile (a) bis (f), umfassend eine chemische Gruppe, die an die Proteingruppe geknüpft ist;
(h) einem Derivat des Teils (g), wobei besagte chemische Gruppe eine wasserlösliche Polymergruppe ist;
(i) einem Derivat des Teils (h), wobei besagte wasserlösliche Polymergruppe Polyethylenglycol ist;
(j) einem Derivat des Teils (i), wobei besagte Polyethylenglycolgruppe nur an den N-Terminus besagter Proteingruppe angehängt ist; und
(h) einem OB-Protein, Analog oder Derivat von einem der Teile (a) bis (h) in einem pharmazeutisch annehmbaren Träger.

2. Verwendung nach Anspruch 1, wobei besagter Patient eine erhöhte Konzentration an Serum-Cholesterin hat und die Dosierung des besagten OB-Proteins, Analogs oder Derivats ausreicht, um die Konzentration an Serum-Cholesterin von besagtem Patienten auf normalem Niveau zu halten.

3. Verwendung nach Anspruch 1, wobei besagter Patient eine erhöhte Konzentration an Serum-Triglyceriden hat und die Dosierung des OB-Proteins, Analogs oder Derivats ausreicht, um die Konzentration an Triglycerid in besagtem Patienten auf normalem Niveau zu halten.

4. Verwendung nach Anspruch 1, wobei besagter Patient eine erhöhte Konzentration an arteriellem Plaque bat und die Dosierung des OB-Proteins, Analogs oder Derivats ausreicht, um die Konzentration an arteriellem Plaque in besagtem Patienten auf normalem Niveau zu halten.

5. Verwendung nach Anspruch 4, wobei eine Verringerung des arteriellen Plaques zu einer Behandlung von Bluthochdruck führt.

6. Verwendung nach Anspruch 1, wobei besagter Patient gegenwärtig oder zuvor Gallensteine gehabt hat und die Dosierung des OB-Proteins, Analogs oder Derivats ausreicht, um die Bildung von zusätzlichen Gallensteinen zu verhindern oder zu verringern.

## Revendications

1. Utilisation d'une protéine OB, d'un analogue ou dérivé de celle-ci pour la fabrication d'un médicament pour réduire le taux de lipides sanguins chez un patient non obèse, ou pour maintenir un taux réduit de lipides sanguins chez un patient non obèse ayant un taux élevé de lipides sanguins, où la protéine OB, l'analogue ou le dérivé de celle-ci est dans une quantité suffisante pour réduire ou maintenir des taux réduits desdits lipides sanguins, mais insuffisante pour entraîner une perte de poids, et où ladite protéine OB, l'analogue ou le dérivé de celle-ci est choisi parmi :
(a) la séquence d'acides aminés 1-146 comme indiqué dans la SEQ ID N° 2 ou la SEQ ID N° 4 ;
(b) la séquence d'acides aminés 1-146 comme indiqué dans la SEQ ID N° 4 ayant un résidu de lysine à la position 35 et un résidu d'isoleucine à la position 74 ;
(c) la séquence d'acides aminés de sous-partie (b) ayant un acide aminé différent substitué dans une ou plusieurs des positions suivantes (en utilisant la numérotation selon la SEQ ID N° 4 et en conservant la même numérotation même en l'absence d'un résidu de glutaminyl à la position 28) : 4, 32, 33, 35, 50, 64, 68, 71, 74, 77, 78, 89, 97, 100, 102, 105, 106, 107, 108, 111, 118, 136, 138, 142 et 145 ;
(d) la séquence d'acides aminés de sous-parties (a), (b) ou (c) éventuellement n'ayant pas un résidu de glutaminyl à la position 28 ;
(e) la séquence d'acides aminés de sous-parties (a), (b), (c) ou (d) ayant un résidu de méthionyl à la terminaison N ;
(f) un analogue de la protéine OB tronqué, choisi parmi : (en utilisant la numérotation de la SEQ ID N° 4) :
(i) les acides aminés 98-146
(ii) les acides aminés 1-32
(iii) les acides aminés 40-116
(iv) les acides aminés 1-99 et 112-146
(v) les acides aminés 1-99 et 112-146 ayant un ou plusieurs des acides aminés 100-111 placés entre les acides aminés 99 et 112 ;
(vi) l'analogue OB tronqué de sous-partie (i) ayant un ou plusieurs des acides aminés 100, 102, 105, 106, 107, 108, 111, 118, 136, 138, 142 et 145 substitués avec un autre acide aminé ;
(vii) l'analogue tronqué de sous-partie (ii) ayant un ou plusieurs des acides aminés 4 et 32 substitués avec un autre acide aminé ;
(viii) l'analogue tronqué de sous-partie (iii) ayant un ou plusieurs des acides aminés 50, 64, 68, 71, 74, 77, 78, 89, 97, 100, 102, 105, 106, 107, 108 et 111 remplacés par un autre acide aminé ;
(vix) l'analogue tronqué de sous-partie (iv) ayant un ou plusieurs des acides aminés 4, 32, 33, 35, 50, 64, 68, 71, 74, 77, 78, 89, 97, 118, 136, 138, 142 et 145 remplacés par un autre acide aminé ;
(x) l'analogue tronqué de sous-partie (v) ayant un ou plusieurs des acides aminés 4, 32, 33, 35, 50, 64, 68, 71, 74, 77, 78, 89, 97, 100, 102, 105, 106, 107, 108, 111, 118, 136, 138, 142 et 145 remplacés par un autre acide aminé ;
(xi) l'analogue tronqué d'une quelconque des sous-parties (i) - (x) ayant un résidu de méthionyl N-terminal ; et
(g) la protéine OB ou l'analogue dérivé d'une quelconque des sous-parties (a) à (f) comportant une fraction chimique reliée à la fraction de protéine;
(h) un dérivé de sous-partie (g) dans laquelle ladite fraction chimique est une fraction de polymère soluble dans l'eau ;
(i) un dérivé de sous-partie (h) dans lequel ladite fraction de polymère soluble dans l'eau est du polyéthylèneglycol ;
(j) un dérivé de sous-partie (i) dans lequel ladite fraction de polyéthylèneglycol est fixée seulement à la terminaison N de ladite fraction de protéine ; et
(h) une protéine OB, un analogue ou un dérivé d'une quelconque des sous-parties (a) à (h) dans un support pharmaceutiquement acceptable.

2. Utilisation selon la revendication 1, dans laquelle ledit patient présente un taux élevé de cholestérol, et ladite posologie de la protéine OB, analogue ou dérivé, est suffisante pour maintenir le taux de cholestérol dudit patient à des taux normaux.

3. Utilisation selon la revendication 1, dans laquelle ledit patient présente un taux élevé de triglycérides, et la posologie de la protéine OB, analogue ou dérivé, est suffisante pour maintenir le taux de triglycérides dudit patient à des taux normaux.

4. Utilisation selon la revendication 1, dans laquelle ledit patient présente un niveau élevé de plaque artérielle, et la posologie de la protéine OB, analogue ou dérivé, est suffisante pour maintenir le niveau de plaque artérielle dudit patient à des niveaux normaux.

5. Utilisation de la revendication 4, dans laquelle la réduction de la plaque artérielle entraîne le traitement d'hypertension.

6. Utilisation de la revendication 1, dans laquelle ledit patient présente actuellement ou a présenté préalablement des calculs biliaires, et la posologie de la protéine OB, analogue ou dérivé, est suffisante pour empêcher ou réduire la formation de calculs biliaires additionnels.
